# EUROPÄISCHE PATENTSCHRIFT

(11) **EP 1 620 146 B1**
(45) Veröffentlichungstag und Bekanntmachung des Hinweises auf die Patenterteilung: **20.02.2008**
(21) Anmeldenummer: 04729069.7
(22) Anmeldetag: 23.04.2004
(51) Int. Cl.: A61M 5/28

(54) **MEHRWEG-BETÄTIGUNGSVORRICHTUNG FÜR EINE STERILE SPRITZE**
MULTIWAY ACTUATING DEVICE FOR A STERILE SYRINGE
DISPOSITIF D'ACTIONNEMENT REUTILISABLE POUR UNE SERINGUE STERILE

(30) Priorität: 05.05.2003 DE 10320225
(43) Veröffentlichungstag der Anmeldung: 01.02.2006
(73) Patentinhaber: L + N Plast Vertriebs GmbH, 82436 Untereglfing (DE)
(72) Erfinder: NEUHOLD, Arnold, 82490 Farchant (DE); BRAEUNINGER-WEIMAR, Willmar, 76414 Rastatt (DE)
(74) Vertreter: HOFFMANN EITLE
(86) Internationale Anmeldenummer: PCT/EP2004/004347
(87) Internationale Veröffentlichungsnummer: WO 2004/098687

(56) Entgegenhaltungen:
- WO-A-20/04028598
- DE-A- 19 948 988
- US-B1- 6 258 068

## Beschreibung

### Gebiet der Erfindung

Die Erfindung betrifft eine Mehrweg-Betätigungsvorrichtung für eine sterile Spritze, insbesondere für eine Spritze deren Nadel von einer Schutzkappe schützend umgeben wird.

Unter einer Mehrweg-Betätigungsvorrichtung versteht man im Sinne der Erfindung ein Gerät, das es durch Betätigung ermöglicht, einen automatischen Einstechvorgang der Nadel durch die Haut in das Gewebe eines Patienten und einen automatischen Injektionsvorgang, bei dem der Inhalt der Spritze in das Gewebe des Patienten injiziert wird, auszulösen.

Ferner ist unter Mehrweg zu verstehen, dass die Betätigungsvorrichtung für mehrere Injektionen verwendet werden kann, wobei lediglich eine herkömmliche Spritze, die in das Gerät einlegbar ist, auszutauschen ist.

### Stand der Technik

Aus dem Stand der Technik sind Betätigungsvorrichtungen bekannt, bei denen eine Nadel automatisch in das Gewebe eines Patienten eingestochen wird und ferner ein Medikament oder Produkt automatisch in das Gewebe injiziert wird. Eine derartige Vorrichtung ist beispielsweise aus WO 94/11041 bekannt. Der hauptsächliche Nachteil dieser Vorrichtung, sowie der meisten bekannten Betätigungsvorrichtungen besteht darin, dass es sich um Wegwerfsysteme handelt. Auch die DE-OS 1 491 696 offenbart eine derartige Vorrichtung, bei der jedoch das Produkt bzw. der Behälter, der das Produkt beinhaltet, vor dem Gebrauch eingesetzt werden muss. Dennoch kann auch diese Vorrichtung nur zur einmaligen Verwendung dienen, weil die Nadel, die auf dem medizinischen Sektor selbstverständlicher Weise sterilen Bedingungen unterliegen muss, fester Bestandteil der Vorrichtung ist. Folglich unterliegen diese Betätigungsvorrichtungen bzw. Injektionsgeräte hinsichtlich der Sterilität ferner einer gesetzlichen Zulassung.

In DE 696 06 993 T2 bzw. EP 0 825 883 B1 ist hingegen ein nachladbarer Autoinjektor offenbart, in den eine Spritze entsprechend einlegbar ist. Folglich ist die Nadel nicht fester Bestandteil des Geräts, so dass dieses keiner Zulassung hinsichtlich der Sterilität bedarf. Bei dieser Betätigungsvorrichtung ist die Nadel der Spritze, wenn sie in die Vorrichtung eingelegt ist, von einer Art Hülse umgeben, die in Einstechrichtung verschieblich ist. Diese Hülse kann jedoch durch ein entsprechendes Sperrelement verriegelt sein. Zum Einstechen der Nadel durch die Haut in das Gewebe eines Patienten muss bei dieser Vorrichtung eine Kraft in Einstechrichtung ausgeübt werden, so dass sich die Hülse, nachdem die Verriegelung aufgehoben wurde, entsprechend verschiebt und die Nadel entsprechend eindringen kann. Dem zu Folge muss bei derartigen Vorrichtungen der Einstechvorgang der Nadel manuell erfolgen. Dies ist jedoch insbesondere in Hinblick auf die Verwendung durch einen Patienten selbst oder ungeschultes Personal nachteilig, da bekanntlich das Einführen einer Nadel in die Haut auf viele Menschen in gewisser Weise abschreckend wirkt und daher diesbezüglich Fehler unterlaufen können.

Ferner ist ein nachladbarer Autoinjektor in der Form einer Pistole aus DE 30 26 318 C2 bekannt.

Die DE 198 21 933 C1 beschreibt andererseits einen Injektor, bei dem das Einstechen der Nadel entweder manuell oder über einen nach dem Aufsetzen des Injektors manuell zu betätigenden Taster automatisch erfolgt.

Ferner offenbart die DE 199 48 988 einen Injektor, bei dem sowohl das Einstechen als auch die Injektion automatisch erfolgen, wobei dieser Vorgang über einen manuell zu betätigenden Taster erfolgt.

### Beschreibung der Erfindung

Dem zu Folge beruht das technische Problem der vorliegenden Erfindung darauf, eine Mehrweg-Betätigungsvorrichtung für eine sterile Spritze bereitzustellen, die nicht zulassungsbeschränkt ist, d. h. die Sterilität der einzulegenden Spritze nicht beeinträchtigt, leicht und einfach zu Hand haben ist und bei der sowohl das Einstechen der Nadel, als auch die Injektion des Inhalts der Spritze voll automatisch erfolgt, ohne dass eine separate Betätigung eines Elements, wie einem Hebel oder Knopf, erfolgt.

Dieses Problem wird erfindungsgemäß durch den im Patentanspruch 1 definierten Gegenstand gelöst. Weitere Vorteilhafte Ausführungsformen sind den Unteransprüchen zu entnehmen.

Insbesondere wird eine Mehrweg-Betätigungsvorrichtung für eine sterile Spritze vorgeschlagen, umfassend einen in einem Gehäuse vorgesehenen vorspannbaren Injektionsmechanismus und eine Auslöseeinrichtung, die mit dem Injektionsmechanismus zusammenwirkt und die Injektion auslöst, wobei die Betätigungsvorrichtung zur Aufnahme der Spritze eine in dem Gehäuse entlang einer Einstechachse translatorisch bewegbare Halterung aufweist. Dadurch, dass die Spritze in einer derartigen Halterung aufgenommen werden kann, wird ermöglicht eine Betätigungsvorrichtung bereitzustellen, bei der der Einstechvorgang als Teil des Injektionsvorgangs automatisch erfolgt, die Betätigungsvorrichtung gleichzeitig jedoch hinsichtlich ihrer Sterilität nicht zulassungsbeschränkt ist. Dies wird insbesondere dadurch erreicht, dass die Spritze, welche ohnehin einer Zulassungsbeschränkung unterliegt, die Injektionsnadel umfasst, in der Halterung in einer Einstechrichtung bewegbar ist, so dass einen automatischer Einstechvorgang ermöglicht ist.

Gemäß der vorliegenden Erfindung umfasst die in die Betätigungsvorrichtung einlegbare sterile Spritze eine Schutzkappe, die die Nadel der Spritze entsprechend schützt. Die Betätigungsvorrichtung umfasst hierfür eine Auslöseeinrichtung die hohlkörperförmig ausgebildet ist und einen Auswurfmitnehmer zum Lösen der Schutzkappe und eine Auswurföffnung zum Auswerfen der Schutzkappe. Dadurch kann ohne dass ein Benutzer mit der Spritze körperlich in Kontakt kommt die Schutzkappe abgezogen werden, wobei die Nadel der Spritze stets, von der Auslöseeinrichtung umgeben, durch die Betätigungsvorrichtung geschützt bleibt. Vorzugsweise umfasst der Auswurfmitnehmer mindestens eine, vorzugsweise zwei Nasen, die mit der Schutzkappe in Eingriff bringbar sind, um diese abzuziehen.

Hauptsächlich aus konstruktiven Gründen ist es vorteilhaft, die Auslöseeinrichtung hülsenförmig auszubilden und die Auswurföffnung zugleich als Durchtrittsöffnung für die Nadel auszugestalten.

Erfindungsgemäß umfasst die Auslöseeinrichtung der Mehrweg-Betätigungsvorrichtung zum Aufsetzen auf einer Oberfläche einen Anlagerandabschnitt über den die Auslöseeinrichtung in einer Einstechrichtung translatorisch beweglich ist, um den Injektionsvorgang auszulösen. Dies weist den Vorteil auf, dass ein Benutzer nicht eine separate Betätigung beispielsweise über einen Hebel oder einen Druckknopf vornehmen muss, sondern lediglich die Betätigungsvorrichtung an der entsprechenden Stelle, an der die Injektion erfolgen soll, aufgesetzt werden muss und durch leichtes drücken in Einstechrichtung sowohl der Einstechvorgang, als auch die Injektion selbst ausgelöst wird.

Vorteilhafterweise umfasst die Auslöseinrichtung einen Auslösevorsprung, der zum Auslösen des Injektionsmechanismus mit einem Auslösemechanismus in Eingriff bringbar ist.

Zu diesem Zweck ist es bevorzugt, dass der Auslösemechanismus eine Doppelklinke umfasst, die drehbar an dem Gehäuse befestigt ist und einerseits mit dem Auslösevorsprung der Auslöseinrichtung und anderseits mit der Halterung in Eingriff bringbar ist. Dadurch wird die Verbindung zwischen der Auslöseinrichtung und der Halterung hergestellt, so dass die Bewegung der Auslöseeinrichtung über die Doppelklinke den Einstechvorgang auslöst. Vorteilhafterweise ist ein Nase der Doppelklinke in einer Richtung federbelastet wegschwenkbar und betätigt in der anderen Richtung einen federbelasteten Auslöseabschnitt, der mit der Halterung in Eingriff steht.

Um die Halterung in dem Gehäuse translatorisch entlang oder parallel zu der Einstechachse zu führen weist die Halterung Führungselemente auf, die bei geschlossenem Gehäuse mit entsprechenden Führungsschienen in dem Gehäuse in Eingriff stehen.

Vorteilhafterweise umfasst der Injektionsmechanismus zum Einstechen der Nadel ein Einstechbetätigungselement, das sich gehäuseseitig abstützt und in vorgespanntem Zustand in Einstechrichtung auf die Halterung wirkt. Das Einstechbetätigungselement ist bevorzugt eine Feder, die sich gehäuseseitig an einem Abstützelement und halterungsseitig an einem Betätigungsabschnitt abstützt.

Ferner umfasst der Injektionsmechanismus eine Injektionseinrichtung, die zum Injizieren des Spritzeninhalts eine Kraft auf einen Kolben der Spritze ausübt. Die Injektionseinrichtung ist vorteilhafterweise in der Halterung angeordnet und stützt sich einerseits an der Halterung, insbesondere einem einfügbaren Zylinderstift ab und wirkt andererseits auf den Kolben der Spritze.

Darüber hinaus ist es bevorzugt, dass der Auslösemechanismus eine an der Halterung drehbar gelagerte federbelastete Klinke mit einem Eingriffsabschnitt umfasst, der im vorgespannten Zustand mit der Injektionseinrichtung in Eingriff steht, so dass die Injektionseinrichtung keine Kraft auf den Kolben der Spritze ausübt, und der Eingriffsabschnitt über die Klinke, die durch die translatorische Bewegung der Halterung mit einem Anschlag des Gehäuses in Eingriff bringbar ist, außer Eingriff mit der Injektionseinrichtung bringbar ist, so dass die Injektionseinrichtung eine Kraft auf den Kolben ausübt. Dadurch kann über die Bewegung der Auslöseeinrichtung, die die Bewegung der Halterung und somit den Einstechvorgang auslöst und über diese Bewegung der Halterung die Injektion in das Gewebe eines Patienten, je nach Anordnung des Anschlags, gleichtzeitig oder zeitversetzt ausgelöst werden.

Um die Betätigungsvorrichtung nach einer Injektion in den Ursprungszustand zurückzusetzen ist vorteilhafterweise eine Vorspanneinrichtung zum Vorspannen des Injektionsmechanismus vorgesehen.

Bei einer Ausführungsform der vorliegenden Erfindung ist die Vorspanneinrichtung mit dem Gehäuse verbunden und in der Halterung translatorisch geführt.

Bei einer weiteren Ausführungsform der vorliegenden Erfindung umfasst das Gehäuse mindestens zwei zueinander verschwenkbare Gehäusehälften und die Vorspanneinrichtung ist mit einer der Gehäusehälften drehbar verbunden und in der Halterung translatorisch geführt, so dass durch Öffnen des Gehäuses, also Verschwenken der Gehäusehälften zueinander der Injektionsmechanismus vorgespannt wird. Dies hat den Vorteil, dass auf Grund der durch das Verschwenken der beiden Gehäusehälften bewirkten Hebelwirkung der Kraftaufwand zum Vorspannen vermindert wird.

Vorteilhafterweise ist die Vorspannvorrichtung ein Zugelement, insbesondere ein Draht. Bevorzugt ist ferner, dass die Vorspannvorrichtung zumindest mit einem Anschlag der Halterung oder der Injektionseinrichtung in Eingriff bringbar ist.

Weiter Bevorzugt weist die Halterung eine Nut auf, die mit der Spritze derart in Eingriff bringbar ist, dass eine translatorische Bewegung der Spritze in und entgegen einer Einstechrichtung verhinderbar und die Spritze trotzdem entnehmbar ist. Dies soll verhindern, dass sich die Spritze während des Einstech- und Injektionsvorgangs entlang der Einstechachse in der Halterung bewegt.

Bevorzugterweise ist die Auslöseinrichtung derart translatorisch entlang einer Einstechachse bewegbar, dass bei einer translatorischen Bewegung in Einstechrichtung die Schutzkappe mittels des Auswurfmitnehmers ablösbar ist, so dass diese durch die Auswurföffnung entnehmbar ist oder durch diese herausfällt und dass der Auslösevorsprung der Auslösevorrichtung mit der Nase der Doppelklinke des Auslösemechanismus in Eingriff kommt, so dass durch eine translatorische Bewegung entgegengesetzt der Einstechrichtung, die durch eine Aufsetzen des Anlagerandabschnitts auf eine Oberfläche bewirkbar ist, der Injektionmechnismus über den Auslösemechanismus auslösbar ist.

Vorteilhafterweise umgibt die Auslöseeinrichtung die Nadel solange, bis der Injektionsvorgang eingeleitet wird.

Es ist bevorzugt, dass wenigstens das Einstechbetätigungselement oder die Injektionseinrichtung eine Feder umfasst.

Darüber hinaus ist es vorteilhaft, dass die Auslöseeinrichtung durch ein mit der Halterung verbundenes Führungselement geführt ist und eine Abstandsfeder, die sich einerseits an der Halterung abstützt und andererseits auf die Auslöseeinrichtung wirkt, die Relativstellung zwischen Auslöseinrichtung und Halterung steuert. Dadurch ist gewährleistet, dass auch beim Anheben der Betätigungsvorrichtung nach der Injektion und beim Herausziehen der Nadel, diese stets durch die Auslöseeinrichtung geschützt bleibt.

### Kurze Beschreibung der Figuren

Im Folgenden wird die vorliegende Erfindung an Hand der begleitenden Zeichnungen rein beispielhaft genauer beschrieben, in denen:
- Fig. 1: (a) eine Seitenansicht ist;
(b) eine Vorderansicht ist;
(c) eine Hinteransicht ist;
(d) eine Unteransicht ist;
(e) eine Draufsicht ist, die jeweils eine Betätigungsvorrichtung gemäß der vorliegenden Erfindung in geschlossenem Zustand zeigen,
- Fig. 2: ein Querschnitt der erfindungsgemäßen Betätigungsvorrichtung mit einer eingelegten Spritze entlang der Linie B-B in Fig. 1(e) ist,
- Fig. 3: (a) eine Seitenansicht ist;
(b) eine Vorderansicht ist;
(c) eine Draufsicht ist;
(d) eine Unteransicht ist;
(e) ein Querschnitt entlang der Linie A-A in Fig. 3(c) ist;
(f) eine Perspektivische Ansicht ist, die jeweils die Halterung einer erfindungsgemäßen Betätigungsvorrichtung zeigen,
- Fig. 4: (a) eine Seitenansicht ist;
(b) eine Vorderansicht ist;
(c) eine Hinteransicht ist;
(d) eine Unteransicht ist;
(e) eine Draufsicht ist;
(f) ein Querschnitt entlang der Linie A-A in Fig. 4(e) ist;
(g) eine perspektivische Ansicht ist, die jeweils einen Teil der zweiten Betätigungseinrichtung der erfindungsgemäßen Betätigungsvorrichtung in zeigen,

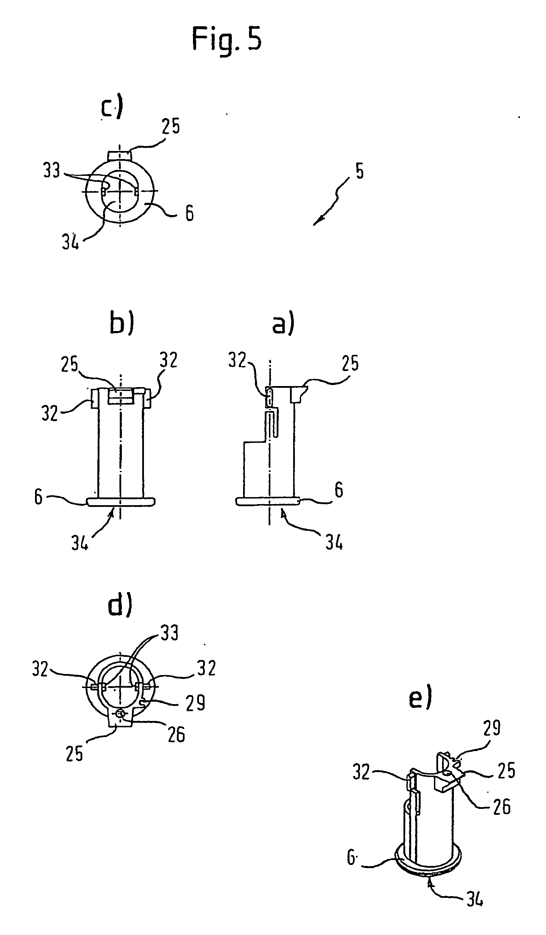

- Fig. 5: (a) eine Seitenansicht ist;
(b) eine Vorderansicht ist;
(c) eine Unteransicht ist;
(d) eine Draufsicht ist;
(e) eine Perspektivische Ansicht ist, die jeweils eine Auslöseeinrichtung der erfindungsgemäßen Betätigungsvorrichtung zeigen,

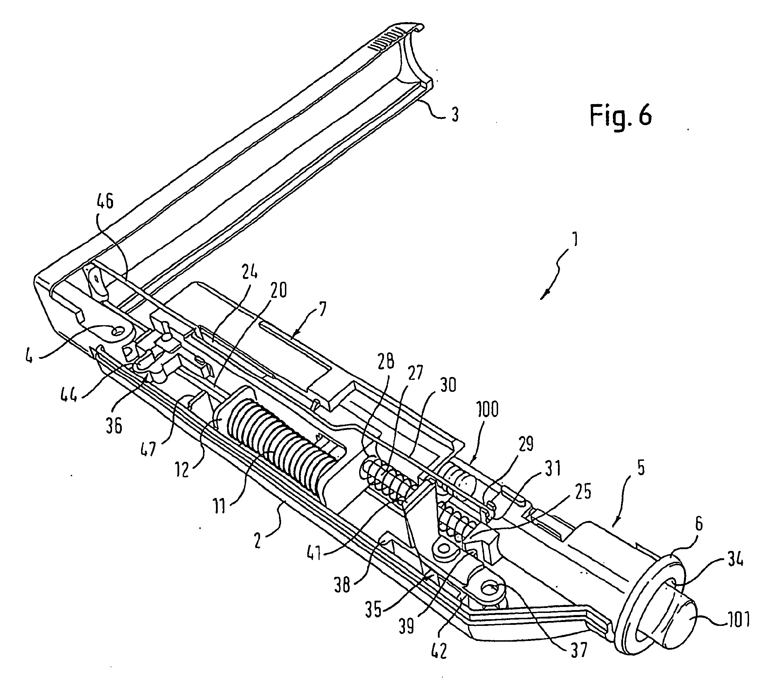

- Fig. 6: die erfindungsgemäße Betätigungsvorrichtung in geöffnetem Zustand mit eingelegter Spritze perspektivisch zeigt.

### Beschreibung der bevorzugten Ausführungsform

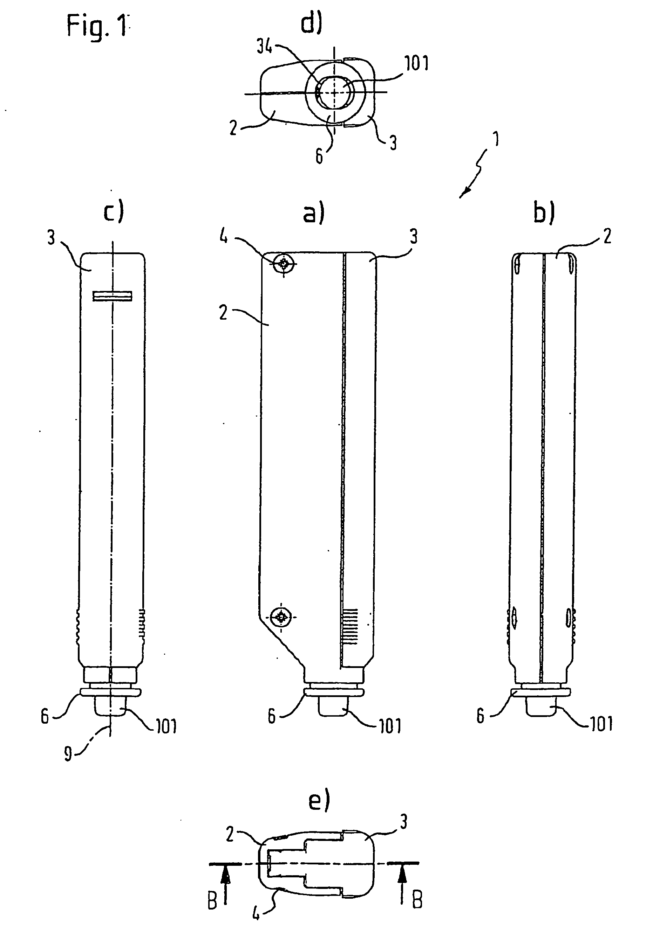

In Fig. 1 ist die erfindungsgemäße Betätigungsvorrichtung 1 dargestellt. Diese umfasst ein Gehäuse, das aus einer ersten Gehäusehälfte 2 und einer zweiten Gehäusehälfte 3 bzw. einem Deckel besteht. Diese beiden Gehäusehälften 2 und 3 sind in einem Punkt 4 gelenkig miteinander verbunden, so dass ein Verschwenken der Gehäusehälften zueinander ermöglicht ist. Wie aus Fig. 1 ersichtlich umgeben die beiden Gehäusehälften 2 und 3 in geschlossenem Zustand eine eingelegte Spritze 100 nahezu vollständig. Lediglich eine Schutzkappe 101, welche die Nadel 102 der Spritze umgibt, steht über einen Anlagerandabschnitt 6 einer Auslöseinrichtung 5 vor. Das Gehäuse muss jedoch nicht notwendigerweise vollständig geschlossen sein. Vielmehr können auch Aussparungen in einer oder beiden Gehäusehälften vorgesehen sein. Ferner könnte beispielsweise in einer der Gehäusehälften, vorzugsweise dem Deckel 3 ein Sichtfenster vorgesehen sein, das einem Benutzer den Blick auf die Spritze 100 ermöglicht.

Die Auslöseinrichtung 5 erstreckt sich durch eine Öffnung, die in geschlossenem Zustand durch die beiden Gehäusehälften 2,3 gebildet wird, nach außen.

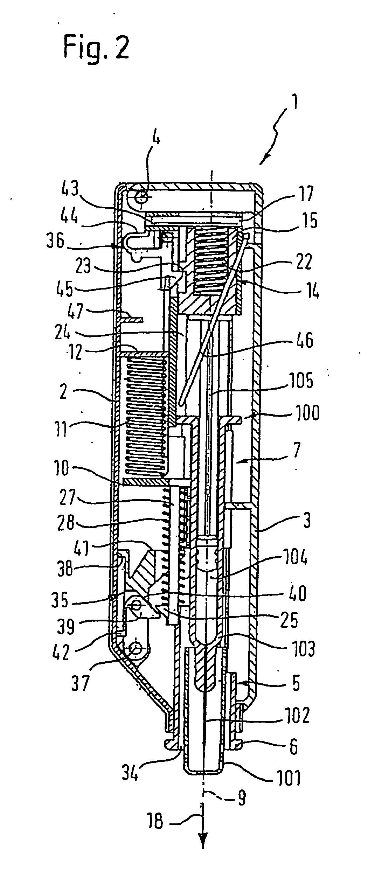

Des Weiteren umfasst die Betätigungsvorrichtung 1, wie Fig. 2 zu entnehmen ist, eine Halterung 7, die dazu dient, die Spritze 100 aufzunehmen und einen Auslösemechanismus der mit einem Injektionsmechanismus zusammenwirkt und den Injektionsvorgang auslöst und über die Auslöseeinrichtung 5 betätigbar ist.

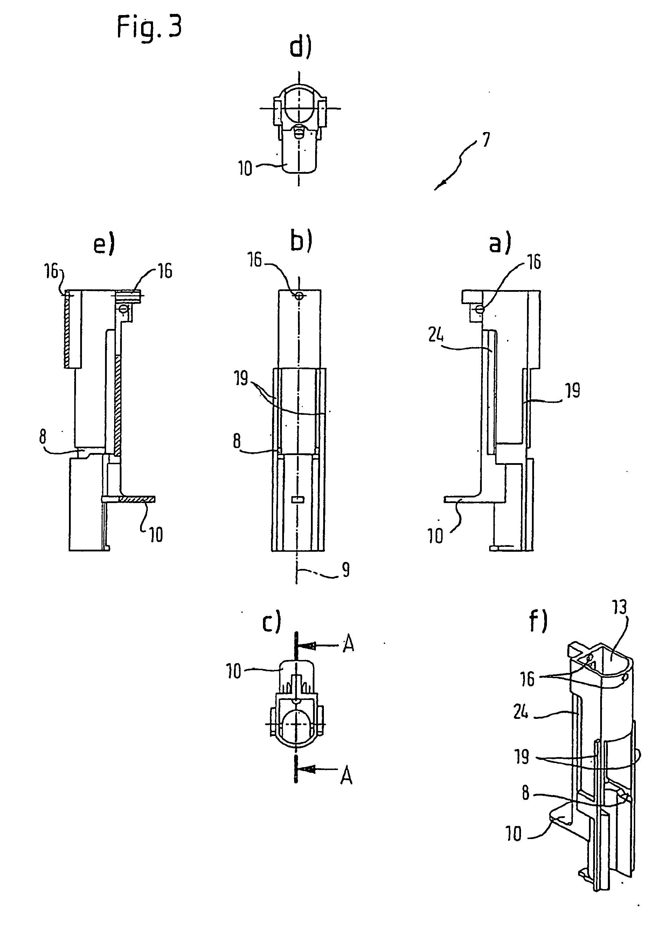

Die Halterung 7 ist in Fig. 3 einzeln dargestellt und umfasst Nuten bzw. Schultern 8, mit denen ein Spritzenkörper 103, der den Spritzeninhalt 104 umfasst und an dem die Nadel 102 angebracht ist, in Eingriff bringbar ist. Dadurch kann eine Bewegung des Spritzenkörpers 103 in seiner Längsrichtung (entlang der Einstechachse 9) verhindert werden, so dass sich der Spritzenkörper 103 sowohl beim Einstechen, als auch während der Injektion nicht verschieben kann.

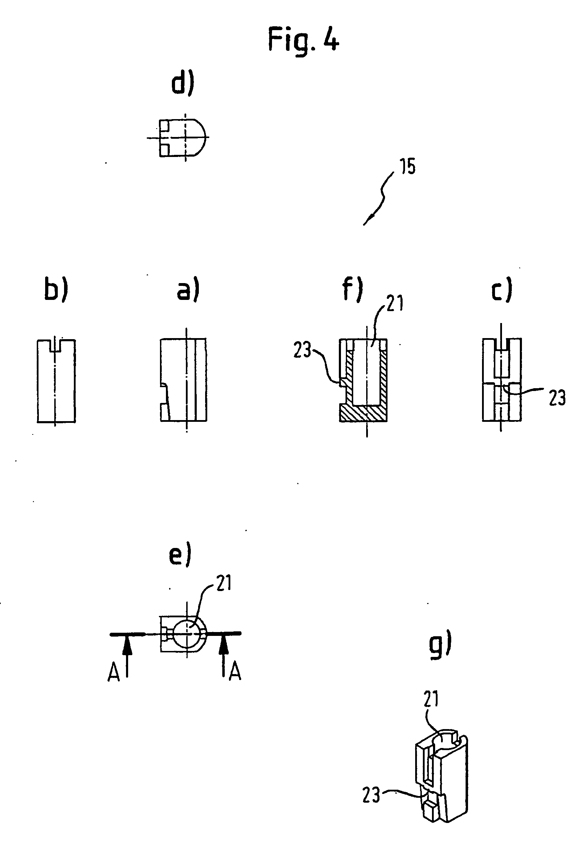

Ferner umfasst die Halterung einen Betätigungsabschnitt 10, auf den ein Einstechbetätigungselement 11 in der Form einer Feder wirkt, die sich gehäuseseitig an einem Abstützabschnitt 12 abstützt. Das Einstechbetätigungselement ist Bestandteil des Injektionsmechanismus. Außerdem umfasst die Halterung 7 einen Hohlraum 13, in den eine Injektionseinrichtung 14 einsetzbar ist. Die Injektionseinrichtung 14 umfasst einen Übertragungskörper 15, der in Fig. 4 einzeln dargestellt ist und eine Injektionsfeder.

Die Halterung weist im Bereich des Abschnitts, in den die Injektionseinrichtung einsetzbar ist Durchgangslöcher 16 auf, in die ein Zylinderstift 17 einführbar ist, um ein herausgleiten der Injektionseinrichtung 14 in axialer Richtung entgegen einer durch den Pfeil 18 angedeuteten Einstechrichtung zu verhindern.

Darüber hinaus umfasst die Halterung 7 Führungselemente 19, die bei geschlossenem Gehäuse in entsprechende Aussparungen 20 in den Gehäusehälften 2 und 3 eingreifen. Dadurch wird eine Führung der Halterung 7 in dem Gehäuse erreicht und nur eine translatorische Bewegung der Halterung 7 entlang bzw. parallel zu einer Einstechachse 9 ermöglicht.

Des Weiteren umfasst die Halterung 7 eine Aussparung 24 zur Führung eines Vorspannhebels.

Wie bereits ausgeführt, ist in die Halterung 7 eine Injektionseinrichtung 14 einsetzbar, die Teil des Injektionsmechanismus ist. Der Übertragungskörper 15 der Injektionseinrichtung 14 ist in Fig. 4 einzeln dargestellt und ist derart ausgestaltet, dass seine Form im Wesentlichen der Form des Hohlraums 13 der Halterung 7 entspricht, in den er einsetzbar ist. In dem Übertragungskörper 15 ist ein zylindrischer Hohlraum 21 vorgesehen, der einseitig, nämlich auf der in Einstechrichtung 18 weisenden Seite, verschlossen ist. In diesen zylindrischen Hohlraum 21 ist eine Injektionsfeder 22 einsetzbar. Der Betätigungskörper 15 ist in der Halterung 7 translatorisch entlang der Einstechachse 9 verschiebbar angeordnet und die Injektionsfeder 22 stützt sich einerseits auf dem Boden des zylindrischen Hohlraums 21 und andererseits an dem in den Durchgangslöchern 16 angebrachten Zylinderstift 17 ab. Ferner umfasst die Injektionseinrichtung 14 einen Verriegelungsvorsprung 23 über den, wie später beschrieben werden wird, ein Verschieben der Injektionseinrichtung 14 verhindert werden kann.

Auch die in Fig. 5 dargestellte Auslöseinrichtung 5 umfasst einen Auslösevorsprung 25. Der Auslösevorsprung 25 ist derart ausgestaltet, dass er im wesentlichen eine Dreiecksform aufweist, also relativ zu der Einstechachse 9 einen schräge Seite und eine im wesentlichen rechtwinklige Seite. In diesem Auslösevorsprung 25 ist ein Durchgangsloch 26 vorgesehen, durch das ein Führungsstift 27 ragt, der mit der Halterung 7 fest verbunden ist. Folglich ist die Auslöseinrichtung 5 über das Durchgangsloch 26 auf dem Führungsstift 27 entsprechend relativ zu der Halterung translatorisch geführt. Darüber hinaus stützt sich eine Abstandsfeder 28, die um den Führungsstift 27 angeordnet ist, einerseits an der Halterung 7 und andererseits an dem Auslösevorsprung 25 der Auslöseinrichtung 5 ab. Diese Abstandsfeder 28 steuert bzw. bestimmt den relativen Abstand zwischen der Halterung 7 und der Auslöseinrichtung 5.

Darüber hinaus ist in einer Nut 29 ein Draht 30 (siehe Fig. 6) entlang bzw. Parallel zu der Einstechachse 9 beweglich geführt. Der Draht 30 ist an dessen Ende gebogen, so dass er einen Endabschnitt 31 aufweist, der senkrecht zur Einstechachse 9 steht. Der Endabschnitt 31 ist mit der Führung bzw. Nut 29 in Eingriff bringbar, um ein Zurücksetzten der Auslöseeinrichtung 5 zu ermöglichen.

Darüber hinaus umfasst die Auslöseeinrichtung 5 zwei Führungselemente 32, die in entsprechenden Führungsschienen zumindest in einer der beiden Gehäusehälften 2,3 geführt sind.

Ferner weist die Auslöseinrichtung 5 einen Auswurfmitnehmer 33, hier in der Form von zwei Nasen auf, die entsprechend hinter die Schutzkappe 101 der Spritze 100 greifen, um mit dieser in Eingriff zu gelangen. Durch eine Auswurföffnung 34, die auch als Durchtrittsöffnung für die Nadel 102 der Spritze 100 dient, kann die Schutzkappe 101 entnommen werden oder sie fällt heraus.

Des Weiteren ist ein Anlagerandabschnitt 6 vorgesehen der entsprechend an der Haut eines Patienten angelegt werden kann, um über den Auslösemechanismus den Injektionsmechanismus auszulösen.

Der Auslösemechanismus umfasst, wie in Fig. 2 dargestellt, eine Doppelklinke 35 und eine Klinke 36.

Die Doppelklinke 35 ist in dem Punkt 37 drehbar am Gehäuse befestigt und über ein flexible Element 38, das sich am Gehäuse 2 abstützt und integral mit der Doppelklinke 35 ausgebildet ist, in einer Richtung federbelastet. Darüber hinaus umfasst die Doppelklinke eine Nase 39. Die Nase 39 ist derart an der Doppelklinke 35 angebracht, dass sie in eine Richtung drehbar und in der anderen Richtung über eine Schräge mit einer komplementären Schulter 40 der Doppelklinke in Eingriff bringbar ist, um einen Auslöseabschnitt 41, der mit der Halterung 7 in Eingriff steht, um den Drehpunkt 37 zu verschwenken. Die Nase 39 ist über ein flexibles Element 42, das integral mit der Nase 39 ausgebildet ist, entgegen der Drehrichtung der Nase 39 federbelastet. Die Nase 39 ist entsprechend mit dem Auslösevorsprung 25 der Auslöseeinrichtung 5 in Eingriff bringbar und derart ausgestaltet, dass sie eine schräge Seite, die entsprechend mit der schrägen Seite des Auslösevorsprungs 25 in Eingriff bringbar ist und eine zur Einstechachse 9 im Wesentlichen senkrechte Seite, die mit der entsprechenden Seite des Auslösevorsprungs 25 in Eingriff bringbar ist, umfasst. Dadurch kann der Auslösevorsprung 25 der Auslöseeinrichtung 5 in der Einstechrichtung 18 in axialer Richtung über die Nase 39 gleiten und liegt in der anderen Richtung an der geraden bzw. zur Einstechachse 9 senkrechten Fläche an.

Die Klinke 36 ist um den Drehpunkt 43 an der Halterung 7 befestigt und stützt sich mit einem integral ausgebildeten flexiblen Element 44 an der Gehäusehälfte 2 ab. Die Klinke umfasst einen Eingriffsabschnitt 45, der mit dem Verriegelungsvorsprung 23 der Injektionseinrichtung 14 in Eingriff bringbar ist. Der Eingriffsabschnitt 45 ist dabei derart ausgestaltet, dass er im wesentlichen eine Dreiecksform aufweist, also relativ zu der Einstechachse 9 einen schräge Seite und eine im wesentlichen rechtwinklige Seite aufweist.
Dadurch wird ermöglicht, dass der Verriegelungsvorsprung 23 der Injektionseinrichtung 14 entgegen der Einstechrichtung 18 in axialer Richtung über den Eingriffsabschnitt 45 verschiebbar ist und in der anderen Richtung an einer geraden senkrecht zur Einstechachse ausgerichteten Fläche des Eingriffsabschnitts 45 anliegt. Bei eingelegter Spritze 100 liegt ein Ende des Kolbens 105 der Spritze 100 an einer Fläche des Übertragungskörpers 15 an.

Wie ferner Fig. 2 zu entnehmen ist, umfasst die Betätigungsvorrichtung 1 eine Vorspanneinrichtung, die einen Vorspannhebel 46 umfasst, der einerseits in der Gehäusehälfte 3 drehbar befestigt und anderseits in der Aussparung 24 in der Halterung 7 translatorisch geführt ist.

Die Gehäusehälfte 2 umfasst einen Anschlag 47, der entsprechend mit der Klinke 36 in Eingriff bringbar ist.

Ferner umfassen beide Gehäusehälften 2 und 3 Versteifungen, die dazu dienen, das Gehäuse, insbesondere während des Spannvorgangs, zu versteifen.

Im Folgenden wird die Verwendung der Vorrichtung beschrieben. Ausgehend von dem in Fig. 2 dargestellten Zustand, in dem die Spritze 100 bereits in die vorgespannte Betätigungsvorrichtung 1 eingelegt ist, wird zum injizieren die Auslöseinrichtung 5 manuell in Einstechrichtung 18 herausgezogen. Dabei kommt der Auswurfmitnehmer 33 in Form der Nasen mit der Schutzkappe 101 der Spritze 100 in Eingriff und löst diese von der Spritze 100 ab. Die Schutzkappe 101 kann dann durch die Auswurföffnung 34 entnommen werden oder fällt durch diese heraus. Durch die Ausgestaltung der Auslöseinrichtung 5 bleibt die Nadel 102 stets verdeckt und geschützt. Beim Bewegen der Auslöseinrichtung 5 in Einstechrichtung 18 kommt die schräge Seite des Auslösevorsprungs 25 mit der schrägen Seite der Nase 39 der Doppelklinke 35 in Eingriff, gleitet über die Nase 39 und drückt diese gegen die Federbelastung durch das flexible Element 42 nach unten. Die Nase 39 verdreht sich bzw. wird verschwenkt, so dass sich der Auslösevorsprung 25 darüber hinwegbewegen kann. Hat sich der Vorsprung 25 über die Nase 39 hinwegbewegt, so schwenkt diese durch die Federkraft des flexiblen Elements 42 in die Ausgangsstellung zurück. In diesem Zustand ist die Betätigungsvorrichtung "scharf" oder, in anderen Worten, betriebsbereit.

Wird daraufhin die Betätigungsvorrichtung 1 mit dem Anlagerandabschnitt 6 auf der Haut eines Patienten aufgesetzt und die Betätigungsvorrichtung 1 leicht in Einstechrichtung 18 gedrückt, so bewegt sich die Auslöseinrichtung 5 entgegen der Einstechrichtung 18 und die senkrecht zur Einstechachse 9 angeordnete Seite des Auslösevorsprungs 25 kommt mit der senkrecht zur Einstechachse angeordneten Seite der Nase 39 in Eingriff. Die Schräge der Nase 39 wiederum kommt mit der dazu komplementären Schulter 40 der Doppelklinke 35 in Eingriff und verschwenkt gegen die Kraft des flexiblen Elements 38 den Auslöseabschnitt 41 um den Drehpunkt 37, so dass der Auslöseabschnitt 41 außer Eingriff mit der Halterung 7 kommt.

Die auf die Halterung 7 wirkende und sich gehäuseseitig abstützende Einstecheinrichtung 11 in Form einer Feder bewegt daraufhin die Halterung 7 mit der Spritze 100 in Einstechrichtung 18 schlagartig nach vorne. Durch diese translatorische Bewegung wird die Nadel 102 durch die Haut in das Gewebe des Patienten eingestochen.

Durch die Bewegung der Halterung 7 in Einstechrichtung 18 bewegt sich auch die Klinke 36, die an der Halterung 7 befestigt ist translatorisch in Einstechrichtung 18. Nach einem vorbestimmten Weg kommt die Klinke 36 mit dem Anschlag 47 des Gehäuses in Eingriff und dreht sich um den Punkt 43. Als Folge kommt der Eingriffsabschnitt 45 der Klinke 36 außer Eingriff mit dem Verriegelungsvorsprung 23 der Injektionseinrichtung 14. Dadurch ist der Übertragungskörper 15 in Einstechrichtung 18 frei beweglich und wird durch die Injektionsfeder 22 in Einstechrichtung 18 bewegt. Dabei stützt sich die Feder 22 einerseits über den Zylinderstift 17 an der Halterung 7 ab und wirkt andererseits auf den Boden des zylindrischen Hohlraums 21 der Injektionseinrichtung 14. Da eine Seite des Übertragungskörpers 15 an dem Kolben 105 der Spritze 100 anliegt wird der Kolben 105 der Spritze 100 entsprechend der Bewegung des Übertragungskörpers 15 bewegt und der Inhalt 106 der Spritze 100 in das Gewebe des Patienten injiziert.

Ist der Injektionsvorgang beendet, so wird die Betätigungsvorrichtung 1 entgegen der Einstechrichtung 18 abgezogen, wodurch die Nadel 102 wieder aus dem Gewebe des Patienten entfernt wird. Damit die Nadel 102 auch bei diesem Vorgang stets geschützt bleibt, drückt die Abstandsfeder 28, die sich an der Halterung 7 abstützt und auf die Auslöseinrichtung 5 wirkt, die Auslöseinrichtung 5 nach vorne, so dass diese die Nadel 102 wieder umgibt und entsprechend schützt.

Um die Spritze 100 zu entnehmen, muss nun die Gehäusehälfte 3 um den Punkt 4 verschwenkt werden. Dadurch wird das Gehäuse geöffnet und der Zugriff auf die Spritze 100 ermöglicht. Beim Verschwenken verschiebt sich der Vorspannhebel 46 in der Führung 24 der Halterung 7 und kommt zuerst mit der Injektionseinrichtung 14, nämlich der mit den Kolben 105 in Verbindung stehenden Seite des Übertragungskörpers 15 in Kontakt. Durch weiteres Öffnen wird die Injektionseinrichtung 14 translatorisch entgegen der Einstechrichtung 18 verschoben, schiebt sich über die Schräge des Eingriffabschnitts 45 der Klinke 36, die, nach dem der Verriegelungsvorsprung 23 der Injektionseinrichtung 14 den Eingriffsabschnitt 45 überwunden hat, auf Grund der Federbelastung durch das flexible Element 44 einrastet und wieder mit dem Verriegelungsvorsprung 23 in Eingriff ist.

Parallel dazu wird die Halterung 7 translatorisch entgegen der Einstechrichtung 18 verschoben, bis der Auslöseabschnitt 41 der Doppelklinke 35 auf Grund der Federbelastung durch das flexible Element 38 einrastet, mit der Halterung also wieder in Eingriff kommt, und eine translatorische Bewegung der Halterung 7 in Einstechrichtung 18 verhindert.

Durch Zurückziehen der Halterung 7 kommt der Endabschnitt 31 des Drahtes 30 mit der Führung 29 der Auslöseinrichtung 5 in Eingriff, so dass der Relativabstand zwischen der Halterung 7 und der Auslöseinrichtung 5 derart festgelegt ist, dass zu dem Zeitpunkt wenn der Auslöseabschnitt 41 wieder einrastet, auch der Auslösevorsprung 25 der Auslöseinrichtung 5 über die Nase 39 der Doppelklinke 35 bewegt wurde.

Es ist anzumerken, dass der Spannvorgang von den verwendeten Federn bzw. Federkräften abhängt. So könnte anstelle des parallelen Spannens der Injektionseinrichtung und der Halterung auch entweder erst die Injektionseinrichtung und dann die Halterung oder umgekehrt gespannt werden, je nachdem wie die Federkräfte der Einstecheinrichtung 11 und der Injektionsfeder 22 gewählt werden.

### Bezugszeichenliste

- 1: Betätigungsvorrichtung
- 2: Gehäusehälfte
- 3: Gehäusehälfte
- 4: Punkt
- 5: Auslöseeinrichtung
- 6: Anlagerandabschnitt
- 7: Halterung
- 8: Schultern
- 9: Einstechachse
- 10: Abstützabschnitt
- 11: Einstecheinrichtung
- 12: Abstützabschnitt
- 13: Hohlraum
- 14: Injektionseinrichtung
- 15: Übertragungskörper
- 16: Durchgangslöcher
- 17: Zylinderstift
- 18: Einstechrichtung
- 19: Führungselemente
- 20: Aussparungen
- 21: Hohlraum
- 22: Injektionsfeder
- 23: Verriegelungsvorsprung
- 24: Aussparung/Führung
- 25: Auslösevorsprung
- 26: Durchgangsloch
- 27: Führungsstift
- 28: Abstandsfeder
- 29: Nut
- 30: Draht
- 31: Endabschnitt
- 32: Führungselemente
- 33: Auswurfmitnehmer
- 34: Auswurföffnung
- 35: Doppelklinke
- 36: Klinke
- 37: Drehpunkt
- 38: Flexibles Element
- 39: Nase
- 40: Schulter
- 41: Auslöseabschnitt
- 42: Flexibles Element
- 43: Drehpunkt
- 44: Flexibles Element
- 45: Eingriffsabschnitt
- 46: Vorspannhebel
- 47: Anschlag
- 100: Spritze
- 101: Schutzkappe
- 102: Nadel
- 103: Spritzenkörper
- 104: Spritzeninhalt
- 105: Kolben
- 106: Inhalt der Spritze

## Patentansprüche

1. Mehrweg-Betätigungsvorrichtung für eine sterile Spritze (100), deren Nadel (102) durch eine Schutzkappe (101) geschützt ist , umfassend
eine zur Aufnahme der Spritze in dem Gehäuse entlang einer Einstechachse (9) translatorisch bewegbare Halterung (7),
einen in einem Gehäuse (2,3) vorgesehenen vorspannbaren Injektionsmechanismus zum automatischen Einstechen der Nadel und zur Injektion, und
eine Auslöseeinrichtung (5), die mit dem Injektionsmechanismus zusammenwirkt und das Einstechen der Nadel und die Injektion beim Aufsetzen auf eine Oberfläche auslöst, **dadurch gekennzeichnet, dass** die Auslöseeinrichtung (5) hohlkörperförmig ausgebildet ist und einen Auswurfmitnehmer (33) zum Lösen der Schutzkappe, eine Auswurföffnung (34) zum Auswerfen der Schutzkappe und einen Anlagerandabschnitt (6) zum Aufsetzen auf einer Oberfläche umfasst und entlang einer Einstechachse (9) bewegbar, so dass bei einer Bewegung in Einstechrichtung (18) die Schutzkappe abgelöst und ausgeworfen wird und bei einer Bewegung entgegengesetzt der Einstechrichtung (18) der Injektionsmechanismus betätigt wird.

2. Mehrweg-Betätigungsvorrichtung nach Anspruch 1, **dadurch gekennzeichnet, dass** der Auswurfmitnehmer (33) mindestens eine, vorzugsweise zwei Nasen umfasst, die mit der Schutzkappe (101) in Eingriff bringbar sind.

3. Mehrweg-Betätigungsvorrichtung nach Anspruch 1 oder 2, **dadurch gekennzeichnet, dass** die Auslöseeinrichtung (5) hülsenförmig ausgebildet ist und die Auswurföffnung (34) zugleich eine Durchtrittsöffnung für die Nadel bildet.

4. Mehrweg-Betätigungsvorrichtung nach einem der Ansprüche 1 bis 3, **dadurch gekennzeichnet, dass** die Auslöseinrichtung (5) einen Auslösevorsprung (25) umfasst, der zum Auslösen des Injektionsmechanismus mit einem Auslösemechanismus in Eingriff bringbar ist.

5. Mehrweg-Betätigungsvorrichtung nach einem der Ansprüche 1 bis 4, **dadurch gekennzeichnet, dass** der Auslösemechnismus eine Doppelklinke (35) umfasst, die drehbar an dem Gehäuse (2) befestigt ist und einerseits mit dem Auslösevorsprung (25) der Auslöseinrichtung (5) und anderseits mit der Halterung (7) in Eingriff bringbar ist.

6. Mehrweg-Betätigungsvorrichtung nach Anspruch 5, **dadurch gekennzeichnet, dass** ein Nase (39) der Doppelklinke (35) in einer Richtung federbelastet wegschwenkbar ist und in der anderen Richtung einen federbelasteten Auslöseabschnitt (41) betätigt.

7. Mehrweg-Betätigungsvorrichtung nach einem der Ansprüche 1 bis 6, **dadurch gekennzeichnet, dass** die Halterung (7) Führungselemente (19) aufweist, die mit entsprechenden Führungsschienen (20) in dem Gehäuse (2) in Eingriff stehen.

8. Mehrweg-Betätigungsvorrichtung nach einem der Ansprüche 1 bis 7, **dadurch gekennzeichnet, dass** der Injektionsmechanismus zum Einstechen der Nadel (102) ein Einstechbetätigungselement (11) umfasst, das sich gehäuseseitig (2,12) abstützt und in vorgespanntem Zustand in Einstechrichtung (18) auf die Halterung (7,10) wirkt.

9. Mehrweg-Betätigungsvorrichtung nach einem der Ansprüche 1 bis 8, **dadurch gekennzeichnet, dass** der Injektionsmechanismus eine Injektionseinrichtung (14) umfasst, die zum Injizieren des Spritzeninhalts (104) eine Kraft auf einen Kolben (105) der Spritze (100) ausübt.

10. Mehrweg-Betätigungsvorrichtung nach Anspruch 9, **dadurch gekennzeichnet, dass** die Injektionseinrichtung (14) in der Halterung (7) angeordnet ist und sich einerseits an der Halterung (7, 17) abstützt und andererseits auf den Kolben (105) der Spritze (100) wirkt.

11. Mehrweg-Betätigungsvorrichtung nach einem der Ansprüche 9 oder 10, **dadurch gekennzeichnet, dass** der Auslösemechanismus eine an der Halterung (7) drehbar gelagerte federbelastete Klinke (36) mit einem Eingriffsabschnitt (45) umfasst, der im vorgespannten Zustand mit der Injektionseinrichtung (14) in Eingriff steht, so dass die Injektionseinrichtung (14) keine Kraft auf den Kolben (105) der Spritze (100) ausübt, und der Eingriffsabschnitt über die Klinke, die durch die translatorische Bewegung der Halterung mit einem Anschlag (47) des Gehäuses (3) in Eingriff bringbar ist, außer Eingriff mit der Injektionseinrichtung bringbar ist, so dass die Injektionseinrichtung eine Kraft auf den Kolben (105) ausübt.

12. Mehrweg-Betätigungsvorrichtung nach einem der Ansprüche 1 bis 11, **dadurch gekennzeichnet, dass** die Betätigungsvorrichtung eine Vorspanneinrichtung (46) zum Vorspannen des Injektionsmechanismus umfasst.

13. Mehrweg-Betätigungsvorrichtung nach Anspruch 12, **dadurch gekennzeichnet, dass** die Vorspanneinrichtung (46) mit dem Gehäuse (3) verbunden ist und in der Halterung (7) translatorisch geführt (24) ist.

14. Mehrweg-Betätigungsvorrichtung nach Anspruch 12, **dadurch gekennzeichnet, dass** das Gehäuse mindestens zwei zueinander verschwenkbare Gehäusehälften (2,3) aufweist, die Vorspanneinrichtung (46) mit einer der Gehäusehälften (3) drehbar verbunden und in der Halterung (7) translatorisch geführt (24) ist.

15. Mehrweg-Betätigungsvorrichtung nach einem der Ansprüche 12 bis 14, **dadurch gekennzeichnet, dass** die Vorspannvorrichtung (46) ein Zugelement, vorzugsweise ein Draht ist.

16. Mehrweg-Betätigungsvorrichtung nach einem der Ansprüche 12 bis 15, **dadurch gekennzeichnet, dass** die Vorspannvorrichtung (46) zumindest mit einem Anschlag der Halterung (7) oder der Injektionseinrichtung (14) in Eingriff bringbar ist.

17. Mehrweg-Betätigungsvorrichtung nach einem der Ansprüche 1 bis 16, **dadurch gekennzeichnet, dass** die Halterung (7) eine Nut (8) aufweist, die mit der Spritze (100) derart in Eingriff bringbar ist, dass eine translatorische Bewegung der Spritze (100) in und entgegen einer Einstechrichtung (18) verhinderbar ist und die Spritze trotzdem entnehmbar ist.

18. Mehrweg-Betätigungsvorrichtung nach Anspruch 5, **dadurch gekennzeichnet, dass** die Auslöseinrichtung (5) translatorisch entlang einer Einstechachse (9) derart bewegbar ist, dass bei einer translatorischen Bewegung in Einstechrichtung mittels des Auswurfmitnehmers (33) die Schutzkappe (101) ablösbar ist, so dass diese durch die Auswurföffnung (34) entnehmbar ist oder durch diese herausfällt und dass der Auslösevorsprung (25) der Auslösevorrichtung mit der Nase (39) der Doppelklinke (35) des Auslösemechanismus in Eingriff kommt, so dass durch eine translatorische Bewegung entgegengesetzt der Einstechrichtung (18), die durch eine Aufsetzen des Anlagerandabschnitts (6) auf eine Oberfläche bewirkbar ist, der Injektionmechnismus über den Auslösemechanismus auslösbar ist.

19. Mehrweg-Betätigungsvorrichtung nach einem der Ansprüche 1 bis 18, **dadurch gekennzeichnet, dass** die Auslöseeinrichtung (5) die Nadel (102) solange umgibt, bis der Injektionsvorgang eingeleitet wird.

20. Mehrweg-Betätigungsvorrichtung nach einem der Ansprüche 8 bis 19, **dadurch gekennzeichnet, dass** wenigstens das Einstechbetätigungselement (11) oder die Injektionseinrichtung (14) eine Feder umfasst.

21. Mehrweg-Betätigungsvorrichtung nach einem der Ansprüche 1 bis 20, **dadurch gekennzeichnet, dass** die Auslöseeinrichtung (5) durch ein mit der Halterung (7) verbundenes Führungselement (27) geführt ist und eine Abstandsfeder (28), die sich einerseits an der Halterung abstützt und andererseits auf die Auslöseeinrichtung wirkt, die Relativstellung zwischen Auslöseinrichtung und Halterung steuert.

## Claims

1. Reusable actuating device for a sterile syringe (100), the needle (102) of which is protected by a protective cap (101), comprising a mounting (7) which can be moved translationally along an insertion axis (9) in the housing to receive the syringe, an injection mechanism, which can be pretensioned, provided in a housing (2, 3) for automatic insertion of the needle and for injection, and a trigger device (5), which cooperates with the injection mechanism and triggers the insertion of the needle and the injection when placing on a surface, **characterised in that** the trigger device (5) is designed to be like a hollow body and comprises an ejection cam (33) to release the protective cap, an ejection opening (34) to eject the protective cap and a support end section (6) for placing on a surface and can be moved along an insertion axis (9), so that during a movement in insertion direction (18), the protective cap is released and ejected and during a movement counter to the insertion direction (18), the injection mechanism is actuated.

2. Reusable actuating device according to claim 1, **characterised in that** the ejection cam (33) comprises at least one, preferably two, noses, which can be engaged with the protective cap (101).

3. Reusable actuating device according to claim 1 or 2, **characterised in that** the trigger device (5) is designed like a sleeve and the ejection opening (34) at the same time forms a passage opening for the needle.

4. Reusable actuating device according to one of claims 1 to 3, **characterised in that** the trigger device (5) comprises a trigger projection (25) which can be engaged with a trigger mechanism to trigger the injection mechanism.

5. Reusable actuating device according to one of claims 1 to 4, **characterised in that** the trigger mechanism comprises a double pawl (35), which is attached rotatably to the housing (2) and can be engaged on one side with the trigger projection (25) of the trigger device (5) and on the other side with the mounting (7).

6. Reusable actuating device according to claim 5, **characterised in that** a nose (39) of the double pawl (35) can be pivoted away in spring-loaded manner in one direction and actuates a spring-loaded trigger section (41) in the other direction.

7. Reusable actuating device according to one of claims 1 to 6, **characterised in that** the mounting (7) has guide elements (19) which are engaged with corresponding guide rails (20) in the housing (2).

8. Reusable actuating device according to one of claims 1 to 7, **characterised in that** the injection mechanism comprises an insertion actuating element (11) for inserting the needle (102) which is supported on the housing side (2, 12) and acts on the mounting (7,10) in insertion direction (18) in pretensioned state.

9. Reusable actuating device according to one of claims 1 to 8, **characterised in that** the injection mechanism comprises an injection device (14) which exerts a force on a piston (105) of the syringe (100) for injection of the syringe content (104).

10. Reusable actuating device according to claim 9, **characterised in that** the injection device (14) is arranged in the mounting (7) and is supported on the mounting (7, 17) on one side and acts on the piston (105) of the syringe (100) on the other side.

11. Reusable actuating device according to one of claims 9 or 10, **characterised in that** the trigger mechanism comprises a spring-loaded pawl (36) rotatably mounted on the mounting (7) and having an engagement section (45) which is engaged with the injection device (14) in the pretensioned state, so that the injection device (14) does not exert a force on the piston (105) of the syringe (100), and the engagement section can be disengaged from the injection device via a pawl, which can be engaged with a stop (47) of the housing (3) by the translational movement of the mounting, so that the injection device exerts a force on the piston (105).

12. Reusable actuating device according to one of claims 1 to 11, **characterised in that** the actuating device comprises a pretensioning device (46) to pretension the injection mechanism.

13. Reusable actuating device according to claim 12, **characterised in that** the pretensioning device (46) is connected to the housing (3) and is guided (24) translationally in the mounting (7).

14. Reusable actuating device according to claim 12, **characterised in that** the housing has at least two housing halves (2, 3) which can be pivoted with respect to one another, the pretensioning device (46) is connected rotatably to one of the housing halves (3) and is guided (24) translationally in the mounting (7).

15. Reusable actuating device according to one of claims 12 to 14, **characterised in that** the pretensioning device (46) is a tension element, preferably a wire.

16. Reusable actuating device according to one of claims 12 to 15, **characterised in that** the pretensioning device (46) can be engaged at least with one stop of the mounting (7) or the injection device (14).

17. Reusable actuating device according to one of claims 1 to 16, **characterised in that** the mounting (7) has a groove (8) which can be engaged with the syringe (100) such that a translational movement of the syringe (100) in and counter to an insertion direction (18) can be prevented and the syringe can nevertheless be withdrawn.

18. Reusable actuating device according to claim 5, **characterised in that** the trigger device (5) can be moved translationally along an insertion axis (9) such that during a translational movement in insertion direction by means of the ejection cam (33), the protective cap (101) can be released so that the latter can be withdrawn through the ejection opening (34) or falls through the latter and that the trigger projection (25) of the trigger device engages with the nose (39) of the double pawl (35) of the trigger mechanism, so that due to a translational movement counter to the insertion direction (18), which can be effected by placing the support end section (6) on a surface, the injection mechanism can be triggered via the trigger mechanism.

19. Reusable actuating device according to one of claims 1 to 18, **characterised in that** the trigger device (5) surrounds the needle (102) until the injection process is initiated.

20. Reusable actuating device according to one of claims 8 to 19, **characterised in that** at least the insertion actuating element (11) or the injection device (14) comprises a spring.

21. Reusable actuating device according to one of claims 1 to 20, **characterised in that** the trigger device (5) is guided by a guide element (27) connected to the mounting (7) and a distancing spring (28), which is supported on the mounting on one side and acts on the trigger device on the other side, controls the relative position between trigger device and mounting.

## Revendications

1. Dispositif d'actionnement réutilisable pour une seringue (100) stérile, dont l'aiguille (102) est protégée par un capuchon de protection (101), comprenant :
une fixation (7), déplaçable en translation le long d'un axe d'enfichage (9), pour loger la seringue dans le boîtier,
un mécanisme d'injection, pouvant être précontraint, prévu dans un boîtier (2, 3), pour l'enfichage automatique de l'aiguille et pour l'injection, et
un dispositif de déclenchement (5), qui coopère avec le mécanisme d'injection et qui déclenche l'enfichage de l'aiguille et l'injection lors du placement sur une surface, **caractérisé en ce que**
le dispositif de déclenchement (5) est réalisé en forme de corps creux et comprend un organe d'entraînement éjection (33) pour détacher le capuchon de protection, une ouverture d'éjection (34) pour éjecter le capuchon de protection, et un tronçon placeur (6) pour placement sur une surface et déplaçable le long d'un axe d'enfichage (9), de sorte que, dans le cas d'un déplacement dans la direction d'enfichage (18), le capuchon de protection est détaché et éjecté et, dans le cas d'un déplacement dans le sens inverse au sens d'enfichage (18), le mécanisme d'injection est actionné.

2. Dispositif d'actionnement réutilisable selon la revendication 1, **caractérisé en ce que** l'organe d'entraînement d'éjection (33) comprend au moins un, de préférence deux, ergots susceptibles d'être mis en prise avec le capuchon de protection (101).

3. Dispositif d'actionnement réutilisable selon la revendication 1 ou 2, **caractérisé en ce que** le dispositif de déclenchement (5) est réalisé en forme de douille et l'ouverture d'éjection (34) forme en même temps une ouverture de passage pour l'aiguille.

4. Dispositif d'actionnement réutilisable selon l'une des revendications 1 à 3, **caractérisé en ce que** le dispositif de déclenchement (5) comprend une saillie de déclenchement (25) susceptible d'être mise en prise avec un mécanisme de déclenchement, pour obtenir le déclenchement du mécanisme d'injection.

5. Dispositif d'actionnement réutilisable selon l'une des revendications 1 à 4, **caractérisé en ce que** le mécanisme de déclenchement comprend un cliquet double (35), fixé de façon à pouvoir tourner sur le boîtier (2) et susceptible d'être mis en prise, d'une part, avec la saillie de déclenchement (25) du dispositif de déclenchement (5) et, d'autre part, avec la fixation (7).

6. Dispositif d'actionnement réutilisable selon la revendication 5, **caractérisé en ce qu'**un ergot (39) du cliquet double (35) est susceptible d'être écarté par pivotement sous la sollicitation d'un ressort, dans un premier sens et, dans l'autre sens, actionne un tronçon de déclenchement (41) sollicité par un ressort.

7. Dispositif d'actionnement réutilisable selon l'une des revendications 1 à 6, **caractérisé en ce que** la fixation (7) présente des éléments de guidage (19) mis en prise dans le boîtier (2) avec des glissières de guidage (20) correspondantes.

8. Dispositif d'actionnement réutilisable selon l'une des revendications 1 à 7, **caractérisé en ce que** le mécanisme d'injection pour l'enfichage de l'aiguille (102) comprend un élément d'actionnement pour enfichage (11) prenant appui côté boîtier (2, 12) et, à l'état précontraint, agissant dans le sens d'enfichage (18) sur la fixation (7, 10).

9. Dispositif d'actionnement réutilisable selon l'une des revendications 1 à 8, **caractérisé en ce que** le mécanisme d'injection comprend un dispositif d'injection (14) qui, pour injecter le contenu de la seringue (104), exerce une force sur un piston (105) de la seringue (100).

10. Dispositif d'actionnement réutilisable selon la revendication 9, **caractérisé en ce que** le dispositif d'injection (14) est disposé dans la fixation (7) et, d'une part, prend appui sur la fixation (7, 17) et, d'autre part, agit sur le piston (105) de la seringue (100).

11. Dispositif d'actionnement réutilisable selon l'une des revendications 9 ou 10, **caractérisé en ce que** le mécanisme de déclenchement comprend un cliquet (36) sollicité élastiquement, monté à rotation sur la fixation (7), avec un tronçon d'engagement (45) qui, à l'état précontraint, vient en prise avec le dispositif d'injection (14), de manière que le dispositif d'injection (14) n'exerce aucune force sur le piston (105) de la seringue (100), et le tronçon d'engagement, par l'intermédiaire du cliquet susceptible d'être mis en prise avec une butée (47) du boîtier (3) du fait du déplacement en translation de la fixation, est susceptible d'être mis hors de prise vis-à-vis du dispositif d'injection, faisant que le dispositif d'injection exerce une force sur le piston (105).

12. Dispositif d'actionnement réutilisable selon l'une des revendications 1 à 11, **caractérisé en ce que** le dispositif d'actionnement comprend un dispositif de précontrainte (46) pour la précontrainte du mécanisme d'injection.

13. Dispositif d'actionnement réutilisable selon la revendication 12, **caractérisé en ce que** le dispositif de précontrainte (46) est relié au boîtier (3) et est guidé en translation (24) dans la fixation (7).

14. Dispositif d'actionnement réutilisable selon la revendication 12, **caractérisé en ce que** le boîtier présente au moins deux demi-boîtiers (2, 3) susceptibles de pivoter l'un par rapport à l'autre, le dispositif de précontrainte (46) est relié à rotation à l'une des moitiés de boîtier (3) et est guidé en translation (24) dans la fixation (7).

15. Dispositif d'actionnement réutilisable selon l'une des revendications 12 à 14, **caractérisé en ce que** le dispositif de précontrainte (46) est un élément de traction, de préférence un fil.

16. Dispositif d'actionnement réutilisable selon l'une des revendications 12 à 15, **caractérisé en ce que** le dispositif de précontrainte (46) est susceptible d'être mis en prise au moins avec une butée de la fixation (7) ou du dispositif d'injection (14).

17. Dispositif d'actionnement réutilisable selon l'une des revendications 1 à 16, **caractérisé en ce que** la fixation (7) présente une rainure (8), susceptible d'être mise en prise avec la seringue (100), de manière qu'un déplacement en translation de la seringue (100), dans et à l'inverse d'un sens d'enfichage (18), puisse être empêché et que la seringue puisse malgré cela être prélevée.

18. Dispositif d'actionnement réutilisable selon la revendication 5, **caractérisé en ce que** le dispositif de déclenchement (5) est déplaçable en translation le long d'un axe d'enfichage (9), de manière qu'en cas d'un déplacement en translation dans le sens d'enfichage, au moyen de l'organe d'éjection (33), le capuchon de protection (101) puisse être détaché, de manière que celui-ci puisse être prélevé à travers l'ouverture d'éjection (34) ou bien tombe à travers celle-ci, et **en ce que** la saillie de déclenchement (25) du dispositif de déclenchement vient en prise avec l'ergot (39) du cliquet double (35) du mécanisme de déclenchement, de manière que, par un déplacement en translation, effectué dans le sens opposé au sens d'enfichage (18), qui peut être provoqué par un placement du tronçon placeur (6) sur une surface, le mécanisme d'injection puisse être déclenché par l'intermédiaire du mécanisme de déclenchement.

19. Dispositif d'actionnement réutilisable selon l'une des revendications 1 à 18, **caractérisé en ce que** le dispositif de déclenchement (5) entoure l'aiguille (102) jusqu'à ce que le processus d'injection ait été induit.

20. Dispositif d'actionnement réutilisable selon l'une des revendications 8 à 19, **caractérisé en ce qu'**au moins l'élément d'actionnement par enfichage (11), ou le dispositif d'injection (14), comprend un ressort.

21. Dispositif d'actionnement réutilisable selon l'une des revendications 1 à 20, **caractérisé en ce que** le dispositif de déclenchement (5) est guidé au moyen d'un élément de guidage (27) relié à la fixation (7), et un ressort d'espacement (28), d'une part, prenant appui sur la fixation et, d'autre part, agissant sur le dispositif de déclenchement, commandant le déplacement relatif entre le dispositif de déclenchement et la fixation.
